# EUROPÄISCHE PATENTANMELDUNG

(11) **EP 2 764 850 A1**
(43) Veröffentlichungstag der Anmeldung: **13.08.2014**
(21) Anmeldenummer: 14150498.5
(22) Anmeldetag: 08.01.2014
(51) Int. Cl.: A61F 2/44, A61F 2/30, A61F 2/28

(54) **Halswirbelimplantat für Hunde**

(30) Priorität: 11.02.2013 DE 102013202211
(71) Anmelder: Rita Leibinger GmbH & Co. KG, 78579 Neuhausen ob Eck (DE)
(72) Erfinder: Forterre, Franck, 3012 Bern (CH); Leibinger, Rita, 78570 Mühlheim (DE); Wirth, Enrico, 78166 Donaueschingen (DE)
(74) Vertreter: DREISS Patentanwälte PartG mbB

(57) **Zusammenfassung**

Die Anmeldung betrifft ein Halswirbelimplantat (22) für Hunde mit einem Plattenabschnitt (24) zum Verbinden zweier benachbarter Wirbelkörper (10,12) und einem Zwischenwirbelabschnitt (20), welcher zwischen den beiden Wirbelkörpern die Bandscheibe ersetzt, wobei zur Befestigung des Implantats der Plattenabschnitt für jeden Wirbelkörper wenigstens eine Aufnahme (28) für eine Knochenschraube (26) aufweist.

## Beschreibung

Die Erfindung betrifft ein Halswirbelimplantat für Hunde.

Zur Fixierung von Wirbelkörpern, insbesondere Halswirbelkörpern, und zur Stabilisation der Halswirbelsäule werden üblicherweise Schrauben und Zement (z.B. PMMA) verwendet. Es ist auch bekannt, dass Platten verwendet werden, die mit Schrauben an den Wirbelkörpern befestigt werden. Die Befestigung dieser Implantate erfolgt mittels eines invasiven Zugangs, wobei die Wirbelendplatten zerstört werden. Außerdem besteht bei diesen Implantaten die Gefahr, dass die Schrauben in den Rückenmarkskanal eindringen und Verletzungen des Rückenmarks beziehungsweise der Nerven verursachen. Um eine stabile Distraktion-Fusion zu erreichen, werden oftmals nicht nur ein sondern zwei Implantate verwendet. Hierdurch wird nicht nur die Gefahr einer Rückenmarksverletzung erhöht, sondern der zeitliche Aufwand des chirurgischen Eingriffs wird verlängert und mitunter können die Implantate nur mit Mühe am Wirbelkörper befestigt werden, da im Halswirbelbereich oftmals der hierfür erforderliche Platz fehlt.

Der Erfindung liegt daher die Aufgabe zugrunde, ein Halswirbelimplantat bereitzustellen, welches einfacher an den Wirbelkörpern befestigt werden kann und welches eine gewisse Distraktion der beiden Wirbelkörper zulässt.

Diese Aufgabe wird erfindungsgemäß mit einem Halswirbelimplantat für Hunde dadurch gelöst, dass das Halswirbelimplantat mit einem Plattenabschnitt zum Verbinden zweier benachbarter Wirbelkörper und einem Zwischenwirbelabschnitt versehen ist, welcher zwischen den beiden Wirbelkörpern die Bandscheibe ersetzt, wobei zur Befestigung des Implantats der Plattenabschnitt für jeden Wirbelkörper wenigstens eine Aufnahme für eine Knochenschraube aufweist.

Beim erfindungsgemäßen Implantat werden also die beiden Wirbelkörper miteinander verbunden und versteift und es wird über den Zwischenwirbelabschnitt sichergestellt, dass die beiden Wirbelendplatten nicht aufeinandertreffen. Der Zwischenwirbelabschnitt hält die beiden Wirbelkörper auf einem der Dicke der Bandscheibe entsprechenden Abstand.

Bei einer Weiterbildung der Erfindung ist vorgesehen, dass die Aufnahme eine Bohrung zur Aufnahme und Lagerung des Kopfes der Knochenschraube aufweist und die Achse der Bohrung beziehungsweise der Schraube gegenüber der Ebene des Zwischenwirbelabschnitts einstellbar ist. Hierdurch wird die Möglichkeit geschaffen, den Plattenabschnitt exakt an die Oberflächen der beiden miteinander zu verbindenden Wirbelkörper anzupassen, so dass die Schrauben in der gewünschten Richtung in den Wirbelkörper eingedreht werden können. Dabei kann die Achse manuell mit oder ohne Werkzeug eingestellt werden. Insbesondere wird die Aufnahme des Plattenabschnitts in die gewünschte Richtung gebogen, so dass das Implantat an die ventrale Knochenoberfläche angepasst werden kann.

Für die Fixierung des Cages am Knochen, wird unter zur Hilfenahme einer Bohrlehre Löcher in einem Idealen Winkel zur Befestigung mit Schrauben, vorzugsweise V-Förmig, gebohrt. Die Kompressionslöcher am Implantat sowie der kugelförmige Kopf der Schraube erzeugen eine Kompression der Wirbelkörper um ein besseres Verwachsen zu gewährleisten.

Vorteilhaft weist der Zwischenwirbelabschnitt wenigstens einen Hohlraum auf, wodurch sein Gewicht verringert und er relativ leicht von Knochengewebe durchwachsen werden kann. Auch die Seitenflächen des Zwischenwirbelabschnitts können Durchbrüche aufweisen. Um die Stabilität des Zwischenwirbelabschnitts zu gewährleisten, sind im Hohlraum Stützstege angeordnet. Diese verlaufen vom Zentrum des Zwischenwirbelabschnitts radial nach außen und können ihrerseits ebenfalls durchbrochen sein.

Mit Vorzug ist der Zwischenwirbelabschnitt konkav und konvex oder in kraniokaudaler Richtung bikonvex ausgebildet, wobei eine der Schmalseiten mit dem Plattenabschnitt verbunden ist. Diese Ausgestaltung erlaubt eine ventrale Einführung und Befestigung des Implantats, so dass die eine konvexe Seite des Zwischenwirbelabschnitts in ventraler Richtung und die andere konvexe Seite in dorsaler Richtung zeigt.

Im Implantat ist eine Öffnung mit Gewinde, die zum einen zur Befüllung des Implantat mit Knochenmaterial vorgesehen ist. Das Knochenmaterial fließt durch die angelegten Hohlräume und verteilt sich dabei gleichmäßig. Die Öffnung mit Gewinde dient auch als Verbindung zu einem Führungsstab der die Positionierung des Implantats vereinfacht.

Um eine optimale Verankerung des Zwischenwirbelabschnitts zwischen den beiden Wirbelkörpern zu erreichen, weist der Zwischenwirbelabschnitt an seinen den Knochenendplatten zugewandten Seiten Verankerungselemente in Form von Dornen auf. Diese verhindern ein Verrutschen beziehungsweise ein Wandern des Implantats zwischen den beiden Wirbelkörpern.

Weitere Vorteile, Merkmale und Einzelheiten der Erfindung ergeben sich aus den Unteransprüchen sowie der nachfolgenden Beschreibung, in der unter Bezugnahme auf die Zeichnung ein besonders bevorzugtes Ausführungsbeispiel im Einzelnen beschrieben ist. Dabei können die in der Zeichnung dargestellten sowie in den Ansprüchen sowie in der Beschreibung erwähnten Merkmale jeweils einzeln für sich oder in beliebiger Kombination erfindungswesentlich sein.

In der Zeichnung zeigen:
- Figur 1: eine schematische Darstellung eines an zwei Wirbelkörpern befestigten Implantats;
- Figur 2: eine perspektivische Ansicht des Implantats; und
- Figur 3: eine weitere perspektivische Ansicht des Implantats.

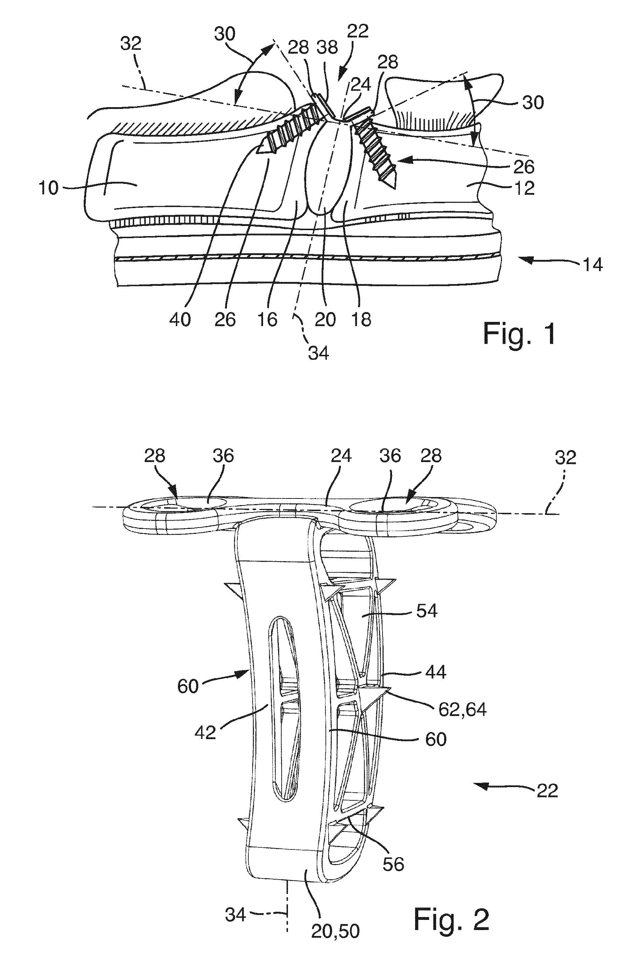

In der Figur 1 ist ein Ausschnitt einer caninen Wirbelsäule dargestellt und es sind von zwei Wirbelkörpern 10 und 12 der ventrale Anteil dieser Wirbelkörper sichtbar. Unterhalb dieser ventralen Anteile befindet sich das Rückenmark 14. Zwischen den beiden Wirbelendplatten 16 und 18 der Wirbelkörper 10 und 12 ist ein Zwischenwirbelabschnitt 20 eines insgesamt mit 22 bezeichneten Halswirbelimplantats angeordnet und stützt die beiden Wirbelendplatten 16 und 18 nach Art einer Bandscheibe gegeneinander ab. Zur Fixierung des Halswirbelimplantats 22 weist dieses noch einen Plattenabschnitt 24 auf, der von mehreren Knochenschrauben 26 durchgriffen ist, wobei die Knochenschrauben 26 im bevorzugten Winkeln in die beiden Wirbelkörper 10 und 12 eingedreht sind. Hierfür sind die Aufnahme 28 um einen Winkel 30 gegenüber der Orthogonalen 32 zur Ebene 34 des Zwischenwirbelabschnitts 20 abgebogen.

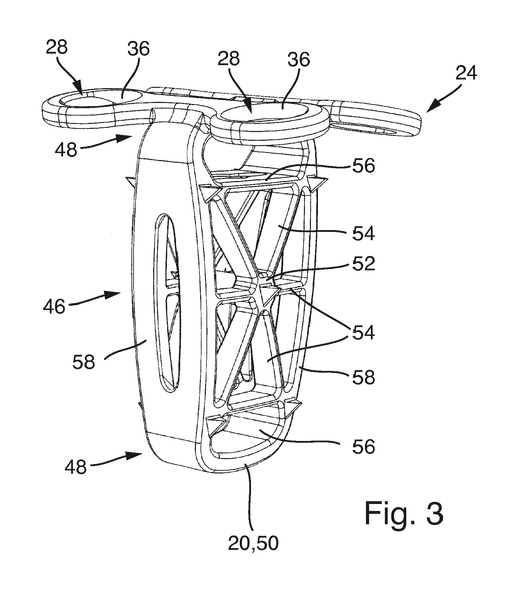

Die Figur 2 zeigt in perspektivischer Ansicht das Halswirbelimplantat 22 und es sind deutlich der Zwischenwirbelabschnitt 20 sowie der Plattenabschnitt 24 erkennbar. Die Aufnahmen 28 befinden sich noch in der Orthogonalen 32 und werden bei Bedarf um den gewünschten Winkel 30 abgebogen. Dies erfolgt z.B. mittels eines speziellen Biegewerkzeugs. Außerdem ist in den Figuren 2 und 3 erkennbar, dass die Aufnahme 38 eine kalottenförmige Einsenkung 36 aufweist, in welcher der Kopf 38 der Knochenschraube 26 liegt. An seinem dem Schraubenschaft 40 zugewandten Ende ist der Kopf 38 teilkugelförmig ausgebildet, so dass die Einsenkung 36 ein Schwenklager für den Kopf 38 bildet. Außerdem ist der Durchmesser des Schraubenschafts 40 so bemessen, dass sich der Kopf 38 in der Einsenkung 36 schwenken und dadurch der Schraubenschaft 40 gegenüber der Ebene der Aufnahme 28 verschwenken kann.

Ferner ist in Figur 2 erkennbar, dass der Zwischenwirbelabschnitt 20 nierenförmig ausgebildet ist und ein Längsabschnitt 42 konkav und der zweite Längsabschnitt 44 konvex ausgeführt ist. Eine bikonvexe Ausführung ist ebenfalls denkbar. Mit einer seiner Schmalseiten ist der Zwischenwirbelabschnitt 20 einstückig mit dem Plattenabschnitt 24 verbunden. Ferner ist erkennbar, insbesondere in Figur 3, dass der Zwischenwirbelabschnitt 20 ballig ausgeführt ist und im mittleren Bereich 46 eine größere Dicke aufweist, als in den Endbereichen 48, und dass der Zwischenwirbelabschnitt 20 als Hohlkörper 50 ausgeführt ist.

Im Hohlkörper 50 befindet sich eine zentrale Achse 52, von welcher radial Stege 54 abgehen und zwei Stege 56 vorgesehen sind, die parallel zum Plattenabschnitt 24 verlaufen. Die Stege 54 und 56 sind ihrerseits durchbrochen, was auch für die konkaven und konvexen Seitenwände 58 zutrifft. Die Durchbrüche bzw. hohle Ausgestaltung des Zwischenwirbelabschnitts 20 begünstigt das Durchwachsen des Implantats 22 mit Knochengewebe.

Schließlich ist noch erkennbar, dass die gewölbte oder ballige Anlagefläche 60, die an den beiden Wirbelendplatten 16 und 18 zu liegen kommt, mit als Dorne 62 ausgebildeten Verankerungselementen 64 bestückt ist. Diese Dorne 62, die spitz zulaufen, dringen in die Wirbelendplatten 16 und 18 ein und verhindern ein Verrutschen des Zwischenwirbelabschnitts 20 und somit des Halswirbelimplantats 22.

## Patentansprüche

1. Halswirbelimplantat (22) für Hunde mit einem Plattenabschnitt (24) zum Verbinden zweier benachbarter Wirbelkörper (10, 12) und einem Zwischenwirbelabschnitt (20), welcher zwischen den beiden Wirbelkörpern (10, 12) die Bandscheibe ersetzt, wobei zur Befestigung des Implantats (22) der Plattenabschnitt (24) für jeden Wirbelkörper (10, 12) wenigstens eine Aufnahme (28) für eine Knochenschraube (26) aufweist.

2. Halswirbelimplantat nach Anspruch 1, **dadurch gekennzeichnet, dass** die Aufnahme (28) eine Bohrung zur Aufnahme und Lagerung des Kopfes (38) der Knochenschraube (26) aufweist und die Achse der Bohrung und/oder der Schraube (26) gegenüber der Ebene (34) des Zwischenwirbelabschnitts (20) einstellbar ist.

3. Halswirbelimplantat nach Anspruch 2, **dadurch gekennzeichnet, dass** die Achse manuell mit oder ohne Werkzeug einstellbar ist.

4. Halswirbelimplantat nach Anspruch 2 oder 3, **dadurch gekennzeichnet, dass** die Bohrung zumindest abschnittsweise kalottenförmig ausgestaltet ist und der Schraubenkopf (38) an seiner dem Schaft (40) zugewandten Seite teilkugelförmig ausgebildet ist.

5. Halswirbelimplantat nach einem der Ansprüche 2 bis 4, **dadurch gekennzeichnet, dass** der Durchmesser der Bohrung um 5 - 50 % größer ist als der Durchmesser des Schafts (40) am kopfseitigen Ende.

6. Halswirbelimplantat nach einem der vorhergehenden Ansprüche, **dadurch gekennzeichnet, dass** der Zwischenwirbelabschnitt (20) wenigstens einen Hohlraum aufweist.

7. Halswirbelimplantat nach Anspruch 6, **dadurch gekennzeichnet, dass** im Hohlraum Stützstege (54, 56) angeordnet sind.

8. Halswirbelimplantat nach einem der vorhergehenden Ansprüche, **dadurch gekennzeichnet, dass** der Zwischenwirbelabschnitt (20) nierenförmig ausgebildet ist und eine der Schmalseiten mit dem Plattenabschnitt (24) verbunden ist.

9. Halswirbelimplantat nach einem der vorhergehenden Ansprüche, **dadurch gekennzeichnet, dass** der Zwischenwirbelabschnitt (20) eine Gitterstruktur aufweist.

10. Halswirbelimplantat nach einem der vorhergehenden Ansprüche, **dadurch gekennzeichnet, dass** der Zwischenwirbelabschnitt (20) an seinen den Wirbelendplatten (16, 18) zugewandten Seiten Verankerungselemente (64) aufweist.

11. Halswirbelimplantat nach Anspruch 10, **dadurch gekennzeichnet, dass** das Verankerungselement (64) als Dorn (62) ausgebildet ist
